# EUROPEAN PATENT SPECIFICATION

(11) **EP 1 986 703 B1**
(45) Date of publication and mention of the grant of the patent: **06.01.2010**
(21) Application number: 07702457.8
(22) Date of filing: 26.01.2007
(51) Int. Cl.: A61L 9/015, A61L 9/20, F24F 3/16, A61L 101/10

(54) **SYSTEM FOR REMOVAL OF AIRBORNE CONTAMINANTS**
SYSTEM ZUR ENTFERNUNG VON LUFTSCHADSTOFFEN
SYSTEME D'ELIMINATION DE CONTAMINANTS ATMOSPHERIQUES

(30) Priority: 27.01.2006 DK 200600128
(43) Date of publication of application: 05.11.2008
(73) Proprietor: Agro Air APS, 8850 Bjerringbro (DK)
(72) Inventor: CARSTEN, Christoffersen, DK-7600 Struer (DK); JÜRGENSEN, Erik, Jessen, DK-4243 Rude (DK)
(74) Representative: Gregersen, Niels Henrik
(86) International application number: PCT/DK2007/000038
(87) International publication number: WO 2007/085262

(56) References cited:
- WO-A-2006/136167
- DE-A1- 10 040 566
- DE-B- 1 246 977
- US-A- 4 141 830
- US-B1- 6 685 825

## Description

The present invention relates to a system for removal of airborne contaminants according to claim 1.

### FIELD OF THE INVENTION

It is generally recognised that airborne pollutants, such as ammonia, bacteria and dust as well as insects and volatile organic compounds having an unpleasant odour inside livestock stables alone and in combination may have a considerable negative impact on the health of production animals and farm workers leading to reduced economy in the livestock production.

A wide range of solutions to these problems has been described including ozone injection to livestock buildings disclosed in US Patent No. 5983834.

Utilisation of ozone and water atomisation to control odour is disclosed in US Patent No. 6076748.

Odour and dust removal equipment using ozone and whirling air stream is disclosed in KR2001069629.

A method and apparatus for producing purified or ozone enriched air to remove contaminants from objects disclosed in WO 00/06209. This method and apparatus relates to the production of purified or ozone enriched air to remove contaminants from objects and is accomplished by a system in which air is drawn in as a stream into the system and flows through ozone generating and germicidal chambers. An ozone generating ultraviolet radiation source disposed within the ozone chamber emits UV radiation having a wavelength approximately 185 nanometers to irradiate the air and generate ozone. The ozonated air enters a germicidal chamber including a germicidal UV radiation source (e.g., emitting radiation having a wavelength of approximately 254 nanometers) that irradiates the ozonated air to destroy contaminants and to catalyse the ozone for enhanced removal of odor causing elements from the air stream. An object of this invention is to remove contaminants from air within a treated space without emitting ozone or ultraviolet radiation into that treated space endangering people and/or animals. However, if the contaminants to be removed comprise particulate matter this will become sticky during ozone treatment and settle on the ultraviolet radiation source with blocking of the emitted radiation as the result. Thus, frequent cleaning of the UV source becomes necessary.

US2004051056 and corresponding US6809326 both discloses an adjustable ozone delivery system for air disinfection comprising an UV light system for treating the air includes an UV tube lamp having a first section for transmitting UV light in a wavelength range that includes maximum ozone production and maximum germicidal activity, and a second section for transmitting UV light in a wavelength range that includes maximum germical activity while excluding the production of ozone; and a movable annualar sleeve for controlling the amount of ozone transmitted by the lamp.

US20040140269A1 discloses an ultraviolet-and-ozone disinfection apparatus providing an improvement on disinfection, which includes a disinfection tank, a mercury UV lamp enclosed by a quartz tube for generating UV light with air trapped between them undergoing UV irradiation to generate ozone, an ozone transmitting tube, a spiral water transmitting tube wrapping around the quartz tube in a spiral manner to achieve sufficient disinfection by mixing ozone in water, a sleeve barrel enclosing the spiral tube. Along the entire flow path, water is exposed to UV radiation to achieve sufficient disinfection and reduce excessive ozone that is harmful to human health.

EP 1362828 A1 and JP 04247294 concerns an ultraviolet lamp used for creating ozone. An ultraviolet lamp is enclosed by a container having an inlet at one end and an outlet at the other end. An air flow containing molecular oxygen is created between the container and the ultraviolet lamp. A portion of the wavelength of the ultraviolet lamp is used for generating ozone. Another portion of the wavelength of the ultraviolet lamp is used to kill micro organisms or disinfect a fluid. The ozone generated may be released in the fluid, further purifying and deodorizing the fluid. The present invention combines the germicidal properties of a ultraviolet lamp with the deodorizing properties of ozone in a single device which may be used to purify water or other fluid.

US4230571 concerns a method and apparatus for the purification of water with ozone and ultraviolet radiation. Oxygen-containing gas, such as air, is directed to flow in a confined path past an ultraviolet radiation source, such as a mercury vapour lamp. The absorption of ultraviolet radiation by oxygen produces ozone which is entrained in the flowing gas. The gas is next mixed with the contaminated water and the mixture of water and ozone is then directed past the same ultraviolet source in a path isolated from the confined path of gas alone. The ozone in the water acts directly to kill bacteria and viruses and to oxidize undesirable compounds in the water. The ultraviolet radiation also acts directly to kill bacteria and viruses in the water. Additionally the ultraviolet radiation acts as a catalyst for the disinfecting and oxidizing action of the ozone, so that water purification occurs much faster than would occur if the ozone were acting alone.

US4141830 describes an apparatus for purifying liquid such as water, in which an ultraviolet light source irradiates air passing through a first chamber surrounding the source, and then irradiates the liquid passing through the second chamber surrounding the first chamber. The air from the first chamber is ozonated by the U.V. light, and this air is bubbled into the water in the second chamber to maximize the purification through simultaneous ultraviolet and ozone exposure.

WO04011127A1 relates to a method of purifying air, a process for manufacture of fertilizer and an apparatus for purifying air by scrubbing with an acid. The apparatus for purifying air contains washing liquid and comprises: at least one air cleaning unit comprising a scrubber in which the air to be purified is washed with the acid containing washing liquid, and from which the purified air and the spent washing liquid is withdrawn; and a washing liquid control unit, arranged in a distance from the air cleaning unit(s), in which the composition of the spent washing liquid withdrawn from the scrubber is readjusted, if desired, by addition of fresh water and fresh acid and withdrawal of a corresponding amount of the spent washing liquid as a product, such as a fertilizer mixture, before the optionally readjusted washing liquid is transferred to and introduced into the scrubber as acid containing washing liquid. Furthermore the invention relates to a building provided with an apparatus for purifying air

DE 10040566 concerns also an apparatus for purifying liquid such as water, where ozone is produced in an air stream flowing inside a tube comprising a UV source. The ozone containing air is mixed with the liquid in an outlet tube. Incoming liquid is at first treated by the UV radiation and later after the liquid has left the radiation area the ozone is pumped into the outlet tube.

It is an object of the invention to provide a system for removal of airborne pollutants in confined spaces, such as a livestock stable. A further object of the invention is to provide a integrated system for the total removal of germs including bacteria, viruses, and fungal spores and flying arthropods present in confined spaces such as livestock production buildings, industrial production buildings, offices, hospitals, schools, etc. A further object of the invention is to overcome the problem of dust accumulation on the ultraviolet light emitting lamp and production of nitrogen containing toxic gases.

This is achieved by the system according to claim 1.

Hereby is achieved an effective system for the removal of airborne contaminants including bacteria, virus, insects, malodour, dust particles, and noxious gases such as NH₃ and H₂S in production buildings such as livestock stables or barns. The method is useful in a system for odour removal where it may be combined with feed-back control. The invention further relates to an ozone production unit useful in the method of the invention. The present system has the advantage of being an integrated system for the total removal of germs including bacteria, viruses, and fungal spores and flying insects, such as flies and mosquitoes, and other small arthropods including spiders and mites present in confined spaces such as livestock production buildings, industrial production buildings, offices, hospitals, schools, etc.

The invention comprises at least one nozzle for spraying water into said chamber for the capture and washing out of airborne particles and gaseous ammonia, and flushing of the outer wall of the oblong production chamber, and where the system is further fitted with a drain outlet for water containing trapped pollutants. It is preferred that the spray water is cold water having a temperature in the range of about 8 to 10°. This will ensure that the spray water may act as a cooling means in addition to acting as a flushing means for the quartz tube and a trapping means for dust particles.

It is preferred that in the system of the invention said sprayed water further contains a diluted acid, such as diluted sulphuric acid. The presence of acid in the sprayed water enables basic compounds such as ammonia to be bound due to salt formation with the acid. An acid, such as sulphuric acid, which forms water soluble salts with basic compounds, is preferred. In this preferred embodiment the system of the invention has the further advantage of being an integrated system for removal of the full range of air borne contaminants from the air in the above mentioned confined spaces, e.g. closed livestock production units and industrial production buildings where dust and optionally also gaseous ammonia are present as air contaminants. A typical level of ammonia in contaminated air inside a livestock stable may range from about 10 to 40 ppm and up to about 200 ppm. In the context of the present invention a full range of air borne contaminants include organic vapours, such as volatile odourous compounds, toxic gases such as hydrogen sulphide and other sulphureous compounds, gaseous ammonia and the like; air borne bacteria, viruses and fungal spores; organic and inorganic dust particles, flying arthropods including flies, thrips, mosquitoes and spiders.

The present invention relates in another aspect to a system as described above which further comprises a secondary reaction chamber for further ozone mediated reduction of airborne contaminants having a final outlet to the confined space, and where the concentration of ozone in the outlet stream is less than about 0.1 - 0.2 ppm, and a secondary reaction zone where further contaminant reduction takes place and where the ozone concentration is reduced to less than about 0.05 - 0.08 ppm when measured in about 1 meter's distance from the outlet. In this embodiment of the invention said secondary reaction chamber is conveniently made up of appropriate piping that leads the discharged cleaned air from the system back into the confined space. An ozone concentration higher than about 0.05 ppm for further odour and germ reduction can safely be allowed inside said secondary reaction chamber. The secondary reaction zone constitutes the space op to about a distance of about 1 meter from the final outlet where still further contaminant reduction may take place until the discharged treated air has an ozone concentration at the level of about 0.05 ppm considered safe for humans. The only slightly elevated ozone concentrations of said secondary reation zone may contribute to reduction or elimination of arthropods in the confined space.

In a further aspect the invention relates to a system as described above, wherein the radiation emitted from the ultraviolet light tube covers the ultraviolet spectrum from about 280 nm to about 100 nm with at least one spectrum peak in the ozone forming range, preferably at about 184.9 nm and with at least one spectrum peak in the germicidal range, preferably at about 253,7 nm. These radiation ranges enables the dual purpose of ozone generation and germ reduction of the UV light tube.

The ultraviolet light tube is mounted inside a protecting tube. In this way the ozone production chamber is separated from the primary reaction chamber where a higher relative humidity would diminish ozone production. The protecting tube is preferably a quartz tube that reflects the ozone forming short wave UV radiation for increasing the radiation inside the tube, which leads to an increase in the formation of ozone. The quartz tube will permit transmission of the germicidal range UV radiation, thus enabling the full effect of the various peak UV wavelengths generated by the ultraviolet light tube to be utilised in the treatment of contaminated air.

The invention relates in a further aspect to an ozone production chamber for use in a system according to claim 1 comprising a tubular quartz cylinder enclosing at least one ultraviolet light tube capable of emitting radiation in the ultraviolet spectrum from about 280 nm to about 100 nm with at least one spectrum peak in the ozone forming range, preferably at about 184.9 nm and with at least one spectrum peak in the germicidal range, preferably at about 253,7 nm and where the quartz cylinder permits penetration of radiation having its wavelength peak in the germicidal range but does not permit penetration of radiation having its wavelength peak in the ozone forming range. The ozone production chamber is preferably an elongated unit wherein the light tube is firmly fitted and wherein the inlet (22) is integrated and connected to conducting means to secure the exclusive intake of oxygen containing air from outside the chamber.

During cold and humid seasons it is preferred that the system of the invention utilises inlet air from the outside which has been subjected to a drying process to obtain a relative humidity of less than about 35% in order to minimise unwanted production of hydrogenperoxide in the production chamber.

The system of the invention is especially useful when the confined space is a livestock stable.

### DESCRIPTION OF THE DRAWINGS

Fig. 1 is a longitudinal section drawing of one possible embodiment of the invention showing the various components and air streams.
Fig. 2 shows a longitudinal section drawing of an embodiment of the production chamber (14) and its position in the system of the invention.

### Detailed description of the drawings

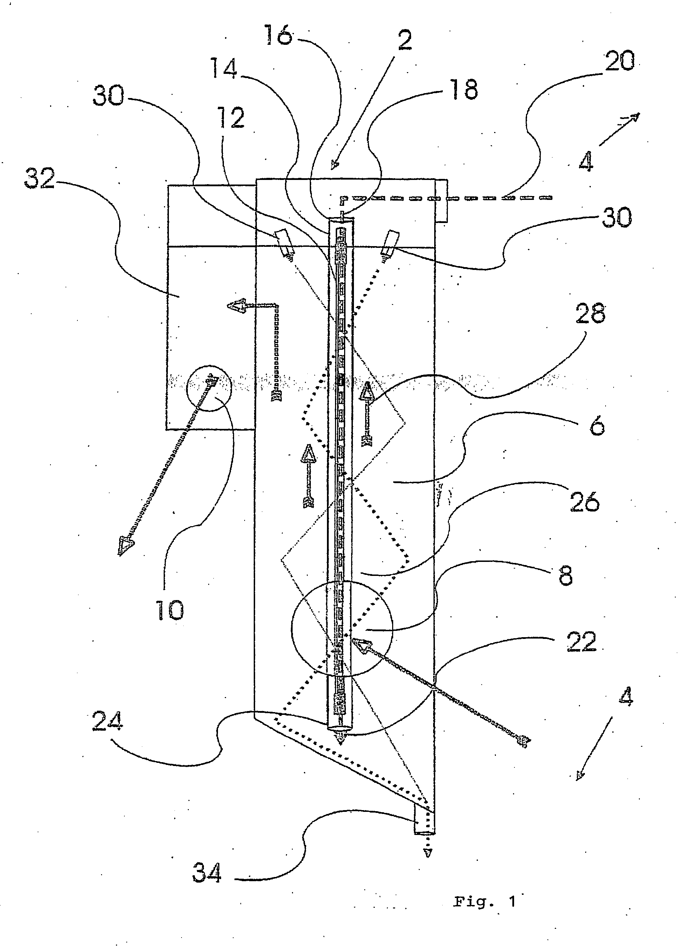

Fig. 1 shows a possible embodiment of a system 2 according to the invention. The system is operating inside a confined space 4, where the system 2 comprises a primary action chamber 6, where the reaction chamber 6 has an inlet 8 for contaminated air coming from the confined space 4. The system 2 furthermore has an outlet 10 also connected to the confined space 4. The chamber 6 comprises an ultraviolet light tube 12 placed inside an oblong ozone production chamber 14. The production chamber 14 has in one end 16 an inlet 18 which inlet 18 is supplied by an oxygen containing air stream 20. The production chamber 14 has an outlet 22 placed in the opposite end 24 also placed inside the reaction chamber 6. Inside the production chamber 14 an ozone containing air stream 26 is generated, which ozone containing air stream 26 is mixed in the primary reaction chamber 6 by contaminated air from the inlet 8 for forming a combined air stream 28. The combined air stream 28 flows in reverse direction upwards outside the production chamber 14. Furthermore, fig. 1 shows nozzles 30 for spraying water or water containing acids inside the chamber 6. A drain outlet 34 is shown for draining the chamber 6. A secondary reaction chamber 32 is connected to the chamber 6 where the outlet 10 is connected to the secondary reaction chamber 32.

In operation a system as shown in fig. 1 is producing ozone-containing air inside the tube 14. At the outlet 22 at the second end 24 of the quartz tube 14 ozone containing air 26 is mixed with the incoming contaminated air and a combined air stream 28 is formed. This air stream 28 is radiated with ultraviolet light so that the air stream 28 is treated both with ozone and ultraviolet light as it is streaming upwards. At the same time the nozzles 30 are spraying water or water which contains e.g. diluted sulphuric acid into the chamber 6 for letting the acid react with the contents of ammonia.

A further positive effect by using the drain nozzles 30 inside the chamber 6 is that the tube 14 on its outside will be continuously washed so that any deposition of dust or other contamination outside the tube 14 will be removed.

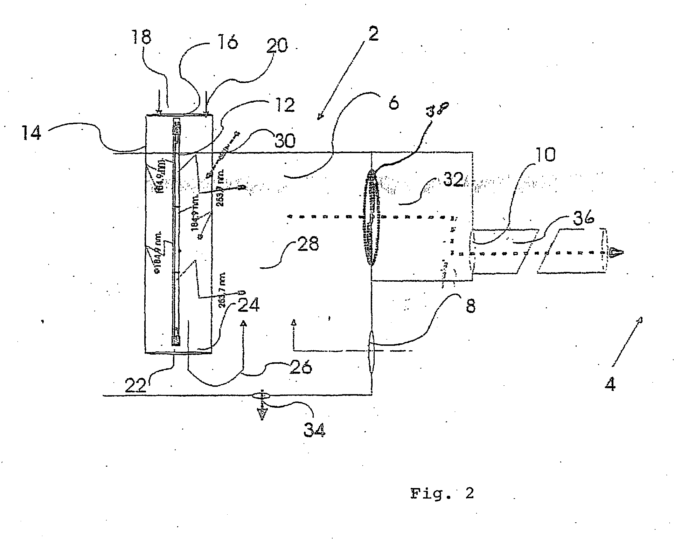

Fig. 2 shows a longitudinal section of a system of a second possible embodiment of the primary production chamber 14 and its position in the system 2. Fig. 2 shows a system 2, which system could be placed inside a confined space 4 but it could also be placed somewhere else where contaminated air has to be cleaned. Fig. 2 shows a reaction chamber 6 which reaction chamber 6 has an inlet 8 and an outlet 10. An ultraviolet light tube 12 is shown inside an oblong ozone production chamber 14 which preferably erably is formed as a quartz tube, the oblong production chamber 14 having in one end 16 an inlet 18 for oxygen containing air stream 20. The oblong production chamber 14 has an outlet 22 positioned in the opposite end 24 inside the primary reaction chamber 6. The ozone containing air stream 26 is mixed inside the chambers with contaminated air coming from the inlet 8 and forming a combined air stream 28. A nozzle 30 for spraying water or water containing diluted sulphuric acid is placed inside the chamber 6 for spraying water or water containing acid for forming a fog which can react with the chemical contents in the chamber 6 and also for cleaning the outside of the oblong production chamber 14 which at the same time is sprayed at the outside. A drain outlet 34 is shown for draining the water or the water containing acid into an outlet. A secondary reaction chamber 32 is shown, to which a tube 36 is connected. Also a ventilator 38 is shown. The ventilator 38 pulls contaminated air 26 through the inlet 8 and through the reaction chamber 6, from where the ventilator pulls the air into the secondary reaction chamber 32 and further through the outlet 10 into piping 36.

The secondary reaction chamber 32 has the effect that an ozone reaction continuous so that the content of ozone is reduced to near a safety level before the air stream leaves the outlet 10.

In a preferred embodiment of the invention the oxygen containing air stream is cooled to a temperature well below about 40 °C in order to prevent the quartz tube from heating to a temperature above 40 °C which could result in reduced ozone production. Further it is preferred that the humidity of the oxygen containing air stream is reduced. In a second possible embodiment of the invention cooling means are used for both reducing the content of humidity and for cooling the oxygen containing air stream.

It is possible to use a very high concentration of oxygen for the production of ozone. Even liquid oxygen could be used after an evaporating process; which process could produce low temperature gaseous oxygen.

### Significance of ozone in the system of the invention

Ozone is a toxic and powerful oxidizing gas having a strong disinfecting and odour reducing effect. In addition, ozone can be produced from oxygen containing gas or air, such as atmospheric air, at the desired site of action, and it decomposes naturally within relatively short time to molecular oxygen leaving non-toxic residual products, depending on temperature, relative humidity, pH and presence of other organic or inorganic matter. Ozone is useful for reduction of air borne pollutants. In the present invention ozone is produced when an oxygen containing air stream, such as an uncontaminated atmospheric air stream, is brought into contact with ultraviolet light. At elevated air humidity there is a risk of generation of hydrogenperoxide instead of ozone, and it is therefore preferred to monitor the atmospheric air humidity and establish a pre-desiccations step before bringing the air in contact with the ultraviolet light tube. Preferably, the oxygen containing air stream has a relative humidity of less than about 35 %. The oxygen containing air is preferably generated from compressed air using a conventional oxygen generator with means for nitrogen removal and cooling means. In this way the oxygen content of the air stream may be elevated in comparison to normal atmospheric air. It is preferred to use clean air with a low content of particles because these will become ionized through contact with ozone and settle on the light tube resulting in loss of effect.

Due to the toxic nature of ozone it is important to minimize its release into the confined space or building to be treated, especially when livestock and humans are present in the building. In this method relatively low concentrations of ozone are produced resulting in a maximum concentration of about 0.1 ppm at a distance of about 1 meter from the outlet. Ozone reacts primarily with C=C and C-H bonds resulting in decomposition of almost all organic substances to carbon dioxide and water. Ozone decomposes pigments and dyes and the majority of odour producing compounds including sulphur compounds and hydrogen sulphide. Ozone oxidizes metal ions. In the system of the invention using ultraviolet light tubes to generate ozone from atmospheric air nitrogen oxides (NOₓ) are not produced or produced only in insignificant amounts.

### Ozone interaction with water and acid

In the present system water and diluted acid, preferably diluted sulphuric acid, is sprayed directly into the chamber (6) through the nozzle (30) for the capture and washing out of airborne particles and gaseous ammonia. Dust particles, insects and organic molecules tend to stick to an ultraviolet light source during decomposition. Therefore it it is preferred, that the outer wall of the oblong ozone production chamber (14) housing the ultraviolet light tube is continually flushed with the sprayed water and diluted acid. Decomposing ozone in the primary reaction chamber (6) generates atomic oxygen that reacts with water droplets or water vapour from the nozzle (30) to form hydroxyl radicals which contributes to pollutant reduction. The diluted acid sprayed into the chamber (6) will react with gaseous ammonia to produce water soluble ammonium salts. The system is fitted with a drain outlet (32) for condensed water from chamber (6), and the water containing trapped pollutants, including odour substances, bacteria, dust, and possibly small insects and spiders and ammonium salts, may be removed through piping to a slurry tank. Due to the use of ozone and sprayed diluted acid, the reaction chamber (6) and its components are made of non-corroding and acid resistant materials.

### Effect of ultraviolet light

In addition to the ozone generating effect of ultraviolet light in the wavelength range of about 100 nm to about 200 nm, ultraviolet light in the wavelength range from about 200 nm to about 280 nm, and preferably with a spectrum peak at approximately 254 nm, has a general germicidal effect including lethal effect on insects and spiders. This is utilized in the present invention where the production chamber (14) comprises at least one ultraviolet light source (12) capable of emitting radiation in both the ozone generating range and in the germicidal range inside a hollow quartz tube, where said tube permits clean atmospheric air to enter through the inlet (18) and become ozonized through the action of ozone forming ultraviolet radiation, and after exiting the production chamber (14) through the outlet (22) to be mixed with contaminated air and sprayed water and diluted acid inside the reaction chamber (6), and where said quartz tube further permits transmission of the germicidal range only of the ultraviolet radiation generated from the light tube (12). The mixture of contaminated air, ozone, sprayed water and sprayed diluted acid comprises a primary reaction mixture where in addition germicidal ultraviolet radiation transmitted through the quartz tube contributes to desinfection.

In a preferred embodiment of the invention the atmospheric air stream following the exit from the passage between ultraviolet light tube (12) and quartz tube (14) is reversed and mixed with contaminated air under simultaneous supply of sprayed water droplets containing diluted acid and then subjected to a passage along the outside of the quartz tube where it is irradiated with ultraviolet light of. 253,7 nm (UV-C) resulting in the formation of atomic oxygen and hydroxyl radicals. This formation takes place with decomposition of a part of the previously formed ozone resulting in a particularly reactive environment. The presence of water and acid, preferably sulphuric acid, will have the effect that a part of the air borne dust particles are washed out while at the same time flushing the quartz tube and prevent it from becoming clogged. The decomposition of pollutants started in the primary reaction chamber will continue when the air stream exits through outlet (10) during the passage through convenient piping to a final discharge to the confined space.

### Monitoring of the system

The air desinfecting and pollutant reducing effect of the system of the invention is dependent on dosage of ultraviolet light and acid as well as air retention time in the system, e.g. retention time in the production chamber (14), the primary reaction chamber (6) and the discharge piping. The ultraviolet dosage can be regulated through control of the number of ultraviolet light tubes in operation and through interaction between the percentage of clean air and recirculated contaminated air from the confined space. The treatment time is controlled through the size of the secondary reaction chambers, e.g. the discharge piping.

In a preferred embodiment of the invention, e.g. when the system is installed in a stable, it is further connected to a feed-back system comprising an electronic nose capable of detecting critical ozone levels and having proper monitoring equipment that can shut down full or partly the ozone generation until the concentration in the stable has returned to acceptable levels. In the same way the concentration of gaseous ammonia may be monitored to regulate the amount of sprayed diluted acid.

### Applicability of the system of the invention

The system is useful in all types of closed livestock stables where the density of production animals leads to air borne pollution resulting in elevated risk of diseases and decreased production economy. The advantages of using the system of the invention include
- reduced pressure of infection from air borne germs and cross contamination
- reduced pressure of infection from the external environment
- reduced amount of insects, spiders and other arthropods in the confined space
- reduced use of antibiotics
- increased fodder utilisation and growth rate
- recirculation of a greater proportion of the total air volume is possible
- reduced costs of heating during the winter season
- economic gain for the farmer
- reduced odour discharge to the environment

## Claims

1. A system (2) for removal of airborne contaminants in a confined space (4) comprising at least one ultraviolet light tube (12), which is enclosed in an oblong ozone production chamber (14), which is formed in a quartz tube, which oblong production chamber (14) in one end (16) has an inlet (18) for an oxygen containing air stream (20) coming exclusively from outside the confined space (4), the system further comprises a primary reaction chamber (6) having an inlet (8) for contaminated air connected to the confined space (4), which chamber (6) has an outlet (10) for treated air connected to the confined space (4) and comprising the ultraviolet light tube (12) for the generation of ozone, in which chamber (6) air coming from the confined space (4) is treated with a combination of UV radiation and ozone, the oblong production chamber (14) has an outlet (22) positioned in the opposite end (24) and inside the primary reaction chamber (6) where the ozone containing air stream (26) is mixed with contaminated air from the inlet (8) to form a combined air stream (28), which is streaming in reversed direction outside the oblong production chamber (14), where the combined air stream (28) is subjected to ultraviolet light transmitted through the wall of the oblong production chamber (14), and where said ultraviolet light having its wavelength spectrum restricted to the germicidal range and through the action of ozone,
**characterized in that**, the system (2) comprises at least one nozzle (30) for spraying water into said chamber for the capture and washing out of airborne particles and gaseous ammonia, and flushing of the outer wall of the oblong production chamber (14), and where the system is further fitted with a drain outlet (34) for water containing trapped pollutants.

2. A system according to claim 1 wherein said water further contains diluted acid, such as diluted sulphuric acid.

3. A system according to any one of the preceding claims further comprising a secondary reaction chamber (32) for further ozone mediated reduction of airborne contaminants having an outlet to the confined space (4), and where the concentration of ozone in the outlet stream is less than about 0.1 - 0.2 ppm, and a secondary reaction zone where further contaminant reduction takes place and where the ozone concentration is reduced to less than about 0.05 - 0.08 ppm when measured in about 1 meter's distance from the outlet.

4. A system according to any one of the preceding claims, wherein said secondary reaction chamber comprises an outlet (36) leading treated air from the primary reaction chamber to a secondary reaction zone within the confined space.

5. A system according to any one of the preceding claims, wherein the radiation emitted from the ultraviolet light tube covers the ultraviolet spectrum from about 280 nm to about 100 nm with at least one spectrum peak in the ozone forming range, preferably at about 184.9 nm and with at least one spectrum peak in the germicidal range, preferably at about 253,7 nm.

6. The system according to claim 5, wherein the ultraviolet light tube is mounted inside a quartz tube.

7. A system according to any one of the preceding claims, wherein an ozone production chamber comprising a tubular quartz cylinder enclosing at least one ultraviolet light tube capable of emitting radiation in the ultraviolet spectrum from about 280 nm to about 100 nm with at least one spectrum peak in the ozone forming range, preferably at about 184.9 nm and with at least one spectrum peak in the germicidal range, preferably at about 253,7 nm and where the quartz cylinder permits penetration of radiation having its wavelength peak in the germicidal range but does not permit penetration of radiation having its wavelength peak in the ozone forming range.

8. The system according to any one of the preceding claims wherein the inlet air from the outside has been subjected to a drying process to obtain a relative humidity of less than 35%.

9. The system according to any one of the preceding claims wherein the confined space is a livestock stable.

## Patentansprüche

1. System (2) zur Beseitigung von schwebenden Schmutzstoffe in einem geschlossenen Raum (4) und umfassend wenigstens ein UV-Rohr (12), das in einer in einem Quarzrohr angeordneten länglichen Ozonbildungskammer (14) eingeschlossen ist, wobei die längliche Ozonbildungskammer (14) in einem Ende (16) einen für einen ausschliesslich von ausserhalb des geschlossenen Raums (4) kommenden sauerstoffhaltigen Luftstrom (20) vorgesehenen Einlass (18) aufweist, wobei das System weiterhin eine mit dem geschlossenen Raum (4) verbundene, mit einem Schmutzlufteinlass (8) versehene primäre Reaktionskammer (6) aufweist, die einen mit dem geschlossenen Raum (4) verbundenen Auslass (10) für behandelte Luft sowie das UV-Rohr (12) zur Ozonerzeugung aufweist, in welcher Kammer (6) die von dem geschlossenen Raum (4) herrührende Luft mit einer Kombination von UV-Strahlung und Ozon behandelt wird, die längliche Ozonbildungskammer (14) weist einen in dem gegenüberliegenden Ende (24) und innerhalb der primären Reaktionskammer (6) angeordneten Auslass (22) auf, wo der ozonhaltige Luftstrom (26) mit Schmutzluft aus dem Einlass (8) zur Bildung eines kombinierten und in umgekehrter Richtung ausserhalb der länglichen Ozonbildungskammer (14) strömenden Luftstroms (28) gemischt wird, wobei der kombinierte Luftstrom (28) von dem durch die Wand des länglichen Ozonbildungskammers (14) transmittierten UV-Licht ausgesetzt wird, und wobei das Wellenlängespektrum des UV-Lichts zu dem keimtötenden Bereich und durch die Wirkung des Ozons beschränkt wird, **dadurch gekennzeichnet, dass** das System (2) wenigstens eine Düse (30) zum Wassersprühen in die Kammer zur Aufnahme und Auswaschen von schwebenden Partikeln und gasförmigem Ammoniak sowie Spülen der Aussenwand der länglichen Ozonbildungskammer (14) aufweist, und wobei das System weiterhin mit einem Ableitungskanal (34) für schmutzstoffhaltiges Wasser versehen ist

2. System nach Anspruch 1, wobei das Wasser weiterhin verdünnte Säure, sowie verdünnte Schwefelsäure, enthält.

3. System nach irgendeinem der vorhergehenden Ansprüchen und weiterhin mit einer für weitere ozonvermittelte Reduktion von schwebenden Schmutzstoffe vorgesehenen sekundären Reaktionskammer (32) mit einem Auslass in die geschlossene Kammer (4), und wobei die Ozonkonzentration im Auslass-Strom weniger als etwa 0.1-0.2 ppm beträgt, sowie einer sekundären Reaktionszone, worin weitere Schmutzstoffreduktion erfolgt, und worin die Ozonkonzentration wenn in einem Abstand von etwa 1 Meter vom Auslass gemessen zu weniger als 0,05-0,08 ppm reduziert ist.

4. System nach irgendeinem der vorhergehenden Ansprüchen, wobei die sekundäre Reaktionskammer einen Auslass (36) aufweist, der behandelte Luft von der primären Reaktionskammer bis zur sekundären Reaktionszone innerhalb der geschlossenen Kammer leitet.

5. System nach irgendeinem der vorhergehenden Ansprüchen, worin die durch das UV-Rohr emittierte Strahlung das UV-Spektrum von etwa 280 nm bis etwa 100 nm mit wenigstens einem Spektrum-Höchstwert in dem Ozonbildungsbereich, vorzugsweise bei etwa 184,9 nm, und mit wenigstens einem Spektrum-Höchstwert in dem keimtötenden Bereich, vorzugsweise bei etwa 253,7 nm, deckt.

6. System nach Anspruch 5, wobei das UV-Rohr innerhalb eines Quarzrohres angeordnet ist.

7. System nach irgendeinem der vorhergehenden Ansprüchen, wobei eine Ozonbildungskammer einen rohrförmigen Quarzzylinder aufweist, der wenigstens ein UV-Rohr einschliesst, das im UV-Spektrum von etwa 280 nm bis etwa 100 nm mit wenigstens einem Spektrum-Höchstwert im Ozonbildungsbereich, vorzugsweise bei etwa 184.9 nm, und mit wenigstens einem Spektrum-Höchstwert im keimtötenden Bereich, vorzugsweise bei etwa 253,7 nm, strahlungsfähig ist, und wobei der Quarzzylinder das Durchdringen der ihren Wellenlänge-Höchstwert in dem keimtötenden Bereich aufweisende Strahlung erlaubt, sondern nicht das Durchdringen der ihren Wellenlänge-Höchstwert im Ozonbildungsbereich aufweisende Strahlung erlaubt.

8. System nach irgendeinem der vorhergehenden Ansprüchen, wobei die von aussen eintretende Luft zum Erreichen einer relativen Feuchtigkeit von weniger als 35% einem Trocknungsvorgang ausgesetzt wird.

9. System nach irgendeinem der vorhergehenden Ansprüchen, wobei der geschlossene Raum ein Viehstall ist.

## Revendications

1. Système (2) d'élimination de polluants atmosphériques dans un espace confiné (4) comprenant au moins un tube à lumière ultraviolette (12) enfermé dans une chambre de production d'ozone oblongue (14) formée d'un tube de quartz, laquelle chambre de production d'ozone oblongue (14) à l'une des extrémités (16) comporte une entrée (18) pour un courant d'air contenant de l'oxygène (20) venant exclusivement de l'extérieur de l'espace confiné (4), le système comprenant encore une chambre de réaction primaire (6) ayant une entrée (8) pour l'atmosphère polluée connectée à l'espace confiné (4), laquelle chambre (6) ayant une sortie (10) pour l'atmosphère traitée connectée à l'espace confiné (4) et comprenant le tube à lumière ultraviolette (12) pour la génération d'ozone, dans laquelle chambre (6) l'atmosphère venant de l'espace confiné (4) étant traitée avec une combinaison de la radiation UV et de l'ozone, la chambre de production oblongue (14) ayant une sortie (22) positionnée à l'extrémité opposée (24) et à l'intérieur de la chambre de réaction primaire (6) dans laquelle le courant d'air contenant de l'ozone (26) étant melangé avec l'atmosphère polluée venant de l'entrée (8) afin de créer un courant d'air mixte (28) dans une direction inverse à l'extérieur de la chambre de production oblongue (14), le courant d'air mixte (28) étant exposé à la lumière ultraviolette transmise à travers la paroi de la chambre de production oblongue (14), et ladite lumière ultraviolette ayant son spectre de longueur d'onde limité au domaine germicide et à travers l'action d'ozone, **caractérisée en ce que** le système (2) comprend au moins une buse (30) pour diffuser l'eau dans ladite chambre afin de capter et laver les particules atmosphériques et l'ammoniac gazeux et arroser la paroi extérieure de la chambre de production oblongue (14), et que le système est encore pourvu d'une sortie d'écoulement (34) pour l'eau contenant des polluants captés.

2. Système selon la revendication 1, dans lequel ladite eau contient encore de l'acide dilué, comme par exemple l'acide sulfurique dilué.

3. Système selon l'une quelconque des revendications précédentes qui comprend encore une chambre de réaction secondaire (32) pour réduire encore avec l'ozone les polluants atmosphériques ayant une sortie à l'espace confiné (4), et la concentration de l'ozone dans le courant de sortie est inférieure à environ 0.1 - 0.2 ppm, et une zone de réaction secondaire dans laquelle une réduction supplémentaire de polluants se produit et dans laquelle la concentration de l'ozone est réduite à moins d'environ 0.05 - 0.08 ppm en mesurant à une distance d'environ 1 mètre de la sortie.

4. Système selon l'une quelconque des revendications précédentes, dans lequel ladite chambre de réaction secondaire comprend une sortie (36) amenant l'atmosphère traitée de la chambre de réaction primaire à une zone de réaction secondaire à l'intérieur de l'espace confiné.

5. Système selon l'une quelconque des revendications précédentes, dans lequel les rayonnements émis du tube à lumière ultraviolette couvrent le spectre ultraviolet à partir d'environ 280 nm à environ 100 nm avec au moins un pic du spectre dans le domaine de la formation de l'ozone, de préférence à environ 184.9 nm et avec au moins un pic du spectre dans le domaine de germicide, de préférence à environ 253,7 nm.

6. Système selon la revendication 5, dans lequel le tube à lumière ultraviolette est monté à l'intérieur d'un tube de quartz.

7. Système selon l'une quelconque des revendications précédentes, dans lequel une chambre de production de l'ozone comprend un cylindre de quartz tubulaire enfermant au moins un tube à lumière ultraviolette pouvant émettre des rayonnements dans le spectre ultraviolet à partir de 280 nm à environ 100 nm avec au moins un pic du spectre dans le domaine de la formation de l'ozone, de préférence à environ 184.9 nm et avec au moins un pic du spectre dans le domaine de germicide, de préférence à environ 253,7 nm, et le cylindre de quartz permet la pénétration des rayonnements avec le pic de la longueur d'onde dans le domaine de germicide mais ne permettant pas la pénétration des rayonnements avec le pic de la longueur d'onde dans le domaine de la formation de l'ozone.

8. Système selon l'une quelconque des revendications précédentes, dans lequel l'air d'entrée venant de l'extérieur a été soumis à un procès de sechage afin d'obtenir une humidité relative de moins de 35%.

9. Système selon l'une quelconque des revendications précédentes, dans lequel l'espace confiné constitue une étable pour les animaux.
